# EUROPEAN PATENT SPECIFICATION

(11) **EP 0 967 916 B1**
(45) Date of publication and mention of the grant of the patent: **14.09.2005**
(21) Application number: 96939790.0
(22) Date of filing: 06.12.1996
(51) Int. Cl.: A61B 5/103, A61B 5/00, A61B 5/05, A45D 44/02

(54) **METHOD AND APPARATUS FOR LOCATING SOFT TISSUE LESIONS**
VERFAHREN UND GERÄT ZUM AUFFINDEN EINER WEICHTEILVERLETZUNG
PROCEDE ET APPAREIL POUR LOCALISER DES LESIONS DES TISSUS MOUS

(43) Date of publication of application: 05.01.2000
(73) Proprietor: Cowie, Jocelyn W., British Columbia V0H 1H0 (CA)
(72) Inventor: Cowie, Jocelyn W., British Columbia V0H 1H0 (CA)
(74) Representative: Nash, David Allan
(86) International application number: PCT/CA1996/000814
(87) International publication number: WO 1998/024365

(56) References cited:
- EP-A- 0 158 336
- EP-A- 0 329 817
- WO-A-91/09566
- FR-A- 2 728 452
- US-A- 4 813 412
- US-A- 4 966 158
- US-A- 5 060 657

## Description

### FIELD

The present method relates to a method and apparatus for assessing and locating soft tissue lesions manifested by pain in the tissue of human beings and animals.

### BACKGROUND

It is known that the electrical resistance of skin is controlled largely through the nervous system. Canadian Patent No. 1,254,269 issued May 16, 1989 to Woodley et al. discloses a diagnostic device based upon resistance measurements of the skin which is used to detect abnormal areas of the body where there is pain or sympathetic disfunction. U.S. Patent No. 4,966,158 issued to Honma et al. discloses a device having two probes which measures the moisture content retained in the skin both in the keratinous layer and also in the deeper layer so as to provide information as to the condition of the skin. Patent Cooperation Treaty Application No. PCT/GB90/01991 discloses a device having a common probe and a reference probe. The measurement of resistance is switched between a common probe applied to an area of skin under test and a reference probe located on the identical area on the other side of the body. A difference in the readings indicates a damaged area of the skin. Thus, known devices measure only one parameter of the skin.

In order to be able to cross correlate different types of measurements of a given area and thus obtain confirmation of the condition and a more accurate diagnosis, it would be useful to be able to measure several different parameters simultaneously. Technically, one could first apply a device to measure resistance to a particular area and then one designed to measure moisture. However, such an approach would be impractical because not only could the condition of the area under test change from one measurement to the other, but positioning the probe on precisely the same area for both measurements would be difficult if not impractical. Secondly, many such measuring devices require measurements to be made using two separate probes applied to two separate but corresponding sides of the body.

Accordingly, it is an object of the invention to provide a method and apparatus for simultaneously measuring at least two different parameters of the skin.

### SUMMARY OF THE INVENTION

Cellular damage can occur due to prolonged stress, which increases build-up of metabolites and tissue ischemia. The latter build-up directly excites pain receptors and causes cellular degeneration or necrosis. Lack of use, poor posture, over use, a blow or hyperextension will cause lesions resulting in inflammation. Inflammation products such as histamine, bradykinin, acids, etc. are released into the capillary bed and produce pain. Pain causes a reflex response of muscle guarding and/or spasms. Such a response leads to immobility and eventual wasting away or atrophy due to loss of the alternating relaxing and contracting of muscles. The muscles provide a circulatory pumping action during normal relaxation and contracting which will ineffective on areas affected by atrophy. Ordinarily, painful areas in the skin are associated with abnormalities such as changes in temperature, and moisture, tenderness, swelling or edema, inflammation, stringiness due to fibrous tissue changes, nodules or small knotted areas, fatigue or lack of tone in the tissues, and metabolite retention characterized by crystal-like formations in the tissue. Such areas of abnormality are conventionally located by a palpative process.

Manual compressions such as applied by accupressure or massage, remove the stimuli causing pain and stopping stimulation of the sweat glands and arterial vessel constriction, the primary cause of pain and degenerative tissue disorders. Such compression and massage are accompanied by the sound of the breaking up processes of metabolite and tissue byproducts. Thus, factors such as moisture, sound, temperature, electrical conductivity, edema are all a function of the condition of the skin and can be used to measure the presence of areas of pain or lesions.

According to the invention there is provided apparatus for diagnosing the skin condition of a human being or animal, which includes a probe for contacting a desired area of skin. The probe has means for measuring the moisture content of the skin at the area and means for measuring the sound generated by the skin at that area, as the probe passes over that area.

A tip of said probe may be rounded and sufficiently small so that it will cause pitting due to edema when pressed against a damaged area of the skin.

Means for measuring the moisture content may be an electrical resistance measuring device for measuring electrical resistance of the skin.

Means for measuring the sound may be a sound meter which is incorporated into the probe having a sound receiving chamber proximate a tip of the probe.

Means for measuring temperature may include a temperature sensor located proximate a tip of the probe.

The means for measuring the moisture content may be an electical resistance measuring device with a contact at a tip of said probe operative to measure the resistance and, hence, moisture content of the skin and the means for measuring the sound may be a sound meter having a sound receiving channel extending to a tip of said probe operative for measuring the sound generated by the skin tissue as said probe passes over a protrusion lesion on the skin.

The temperature sensor located may be a thermocouple located at a tip of said probe for measuring the temperature of the skin contacted by said probe.

A pressure sensor may be located at a tip of the probe and be operative to indicate the force applied to the probe as it passes over the skin.

The pressure sensor may be retractable to accomodate changes in profile of the skin due to lesions and protuberances.

In another aspect of the invention there is provided a method of locating soft tissue lesions which includes passing a probe over the skin of a patient and measuring sound emanating from the skin so as to rapidly locate a pain producing area of the skin and measuring the electrical resistance of the skin at the area. The method further includes re-measuring the resistance and sound of the skin at the area to determine the amount of decrease of the skin lesion.

The method further measures the temperature at the point of contact of the skin with the probe.

### BRIEF DESCRIPTION OF THE DRAWINGS

Further features and advantages will be apparent from the following detailed description, given by way of example, of a preferred embodiment taken in conjunction with.the accompanying drawings, wherein:
FIG. 1 is a sectional elevation view of the probe of an instrument for measuring temperature, sound, moisture and applied force;
FIG. 2 is a top view of the probe shown at a reduced scale from that of Figure 1;
FIG. 3 is a graph of temperature versus position of the probe on the skin along a line through a lesion;
FIG. 4 is a graph of electrical resistance as a function of the probe position on the skin along a line through a lesion;
FIG. 5 is a graph of the sound emitted by the skin during massage with the probe as a function of probe position on the skin along a line through a lesion; and
FIG. 6 is a graph of pressure as a function of the position of the probe on the skin along a line passing through a protrusion lesion

### DETAILED DESCRIPTION WITH REFERENCE TO THE DRAWINGS

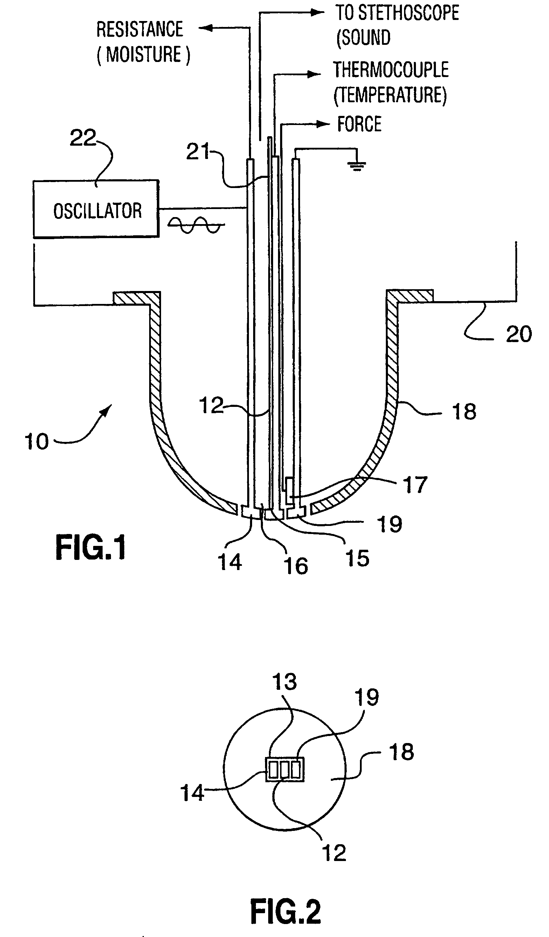

Referring to Figures 1 and 2 there is shown a probe **10** having a generally semi-circular cross-section from the tip **18** back to the casing **20** and an rectangular opening **13** through which there protrudes three sensors **14, 12** and **19.** An open area **16** around the sensors serves to conduct sound up to a sound detector or stethoscope (not shown) of conventional design.

A galvanometer junction **15** is located at the end of sensor **12.** The leads **21** from the galvanometer junction **15** run up to a meter (not shown) which displays the temperature of the junction **15.**

Sensor **19** has a two-fold function. It acts as a ground terminal in conjunction with terminal **14** as well as providing strain to transducer **17** to allow the latter to measure applied force. The applied force provides a useful objective measure of the force being applied with the probe **10.**

In operation, the moisture meter component of the probe **10** is formed by oscillator **22,** conductive element **14,** conductive element **19** and amplifier, filter, rectifier and an analog to digital converter as shown in U.S. Patent No. 4,966,158.

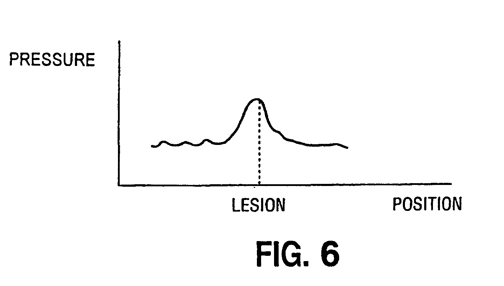

The simultaneous development of signals which correspond to moisture content, temperature and sound allow all three of these factors to be cross correlated to confirm an indicated condition by any one of them and to more accurately define the nature and extent of the condition. The pressure indicator allows a user to monitor and control the amount of pressure being applied. One may determine the pressure required to cause pitting edema, another sign of lesions. As protrusion lesions develop and become fibrous, they create a "speed bump" to the probe **10** as it passes over the area of the protrusion lesion. Applied pressure rises as the pressure sensor on the probe passes over a protrusion lesion as shown in Figure 6. Sensor **19** is permitted to retract slightly to accomodate the change in skin profile presented by a protrusion lesion.

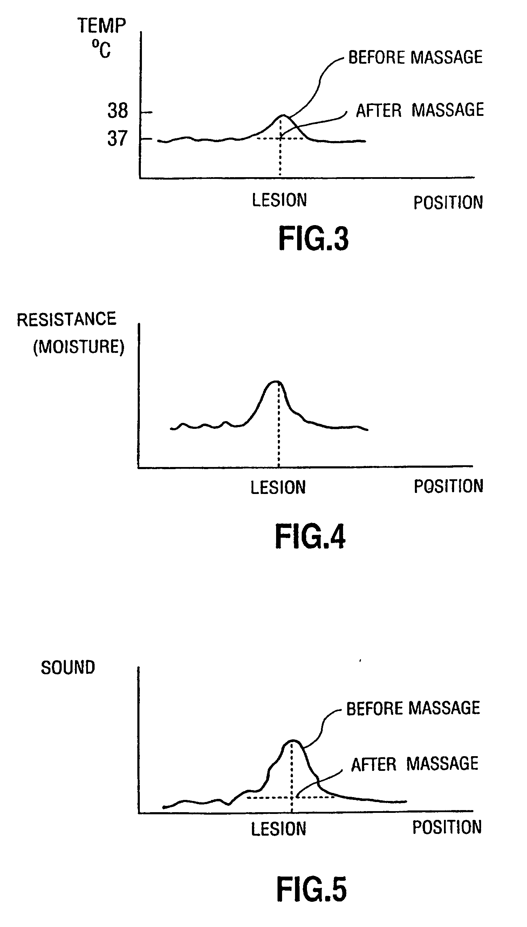

Figures 3 to 5 shows graphs of the readings of temperature, resistance, and sound as one progresses towards, over and then away from a lesion. The same Figures show readings of the same lesion after applying massage (see the dotted lines). Thus, the probe allows the massage therapist to determine the effect of the massage on a lesion to a quantifiable extent. A therapist can use the sound output to rapidly locate suspected damaged areas of the skin and then to confirm the damage using the other readings of temperature, moisture content and resistance. Any extraneous readings in any one or more of the factors of temperature, moisture, and sound can be checked as to their origin by comparing them to the readings for the other factors.

Accordingly, while this invention has been described with reference to illustrative embodiments, this description is not intended to be construed in a limiting sense.

## Claims

1. Apparatus for diagnosing the skin condition of a human being or animal, comprising a probe (10) for contacting a desired area of skin, which comprises means for measuring the moisture content of the skin at said area (14),
**characterized in that** said probe (10) comprises means for measuring the sound (16) generated by the skin tissue at said area as said probe passes over said area.

2. Apparatus according to claim 1, wherein a tip (18) of said probe (10) is rounded and sufficiently small so that it will cause pitting due to edema when pressed against a damaged area of the skin.

3. Apparatus according to claim 1, including means for measuring the temperature (12) of the skin tissue at said area.

4. Apparatus according to claim 1, wherein said means for measuring the moisture content is an electrical resistance measuring device (14) operative to measure the electrical resistance of the skin.

5. Apparatus according to claim 1, wherein said means for measuring the sound is a stethoscope which is incorporated into said probe having a sound receiving chamber (16) proximate a tip (18) of said probe (10).

6. Apparatus according to claim 3, wherein said means for measuring temperature includes a thermocouple (12) located proximate a tip (18) of said probe (10).

7. Apparatus according to claim 1, including means for measuring the force applied to the probe (10) as the probe is passed over the skin.

8. Apparatus according to claim 1, wherein said means for measuring the moisture content is an electrical resistance measuring device (14) with a contact at a tip (18) of said probe (10) operative to measure the resistance and, hence, moisture content of the skin at said area and said means for measuring the sound is a sound meter having a sound receiving channel (16) extending to the tip of said probe operative for measuring the sound generated by the skin tissue as said probe passes over a lesion or protuberance on the skin.

9. Apparatus according to claim 8, including a temperature sensor located at a tip of said probe for measuring the temperature of the skin contacted by said probe.

10. Apparatus according to claim 9, including a pressure sensor located at a tip of said probe operative to indicate the force applied to said probe as it passes over the skin.

11. Apparatus according to claim 10, wherein said pressure sensor is retractable to accommodate changes in profile of the skin due to lesions and protuberances.

12. A method of locating soft tissue lesions, comprising:
(a) providing a probe (10) having means (14,16) for measuring the moisture content of an area of skin tissue and the sound emanating from said area and optionally, pressure of said area as said probe is passed over said area of skin;
(b) passing said probe over the skin of a patient and measuring the moisture content and the sound emanating from said area and optionally, the pressure of said area.

13. A method according to claim 12, including measuring the temperature of said area of the skin with the probe.

14. A method according to claim 12, wherein step (b) is periodically repeated to determine the effect of a massage on the soft tissue lesion.

## Patentansprüche

1. Vorrichtung zur Diagnose des Hautzustandes eines Menschen oder eines Tieres, aufweisend einen Fühler (10) zum Berühren des gewünschten Hautbereiches, welcher Mittel zum Messen des Feuchtigkeitsgehaltes der Haut an diesem Bereich (14) aufweist,
**dadurch gekennzeichnet, dass** der Fühler (10) Mittel zum Messen des Schalls (16) aufweist, welcher durch das Hautgewebe an jenen Bereich erzeugt wird, wenn der Fühler über den Bereich streicht.

2. Vorrichtung nach Anspruch 1, wobei eine Spitze (18) des Fühlers (10) abgerundet und ausreichend klein ist, so dass sie eine Narbenbildung aufgrund von Ödemen bewirkt, wenn sie gegen den geschädigten Bereich der Haut gedrückt wird.

3. Vorrichtung nach Anspruch 1, umfassend Mittel zum Messen der Temperatur (12) des Hautgewebes an dem Bereich.

4. Vorrichtung nach Anspruch 1, wobei das Mittel zum Messen des Feuchtigkeitsgehaltes eine elektrische Widerstandsmessvorrichtung (14) ist, welche dafür ausgelegt ist, den elektrischen Widerstand der Haut zu messen.

5. Vorrichtung nach Anspruch 1, wobei das Mittel zum Messen des Schalls ein Stethoskop ist, welches in den Fühler integriert ist und eine Tonaufnahmekammer (16) neben der Spitze (18) des Fühlers (10) aufweist.

6. Vorrichtung nach Anspruch 3, wobei das Mittel zum Messen der Temperatur ein Thermoelement (12) aufweist, das benachbart einer Spitze (18) des Fühlers (10) liegt.

7. Vorrichtung nach Anspruch 1, umfassend Mittel zum Messen der auf den Fühler (10) ausgeübten Kraft, wenn der Fühler über die Haut geführt wird.

8. Vorrichtung nach Anspruch 1, wobei das Mittel zum Messen des Feuchtigkeitsgehalts eine elektrische Widerstandsmessvorrichtung (14) ist, wobei ein Kontakt an einer Spitze (18) des Fühlers (10) dafür ausgelegt ist, den Widerstand und somit den Feuchtigkeitsgehalt der Haut in dem Bereich zu messen, und wobei das Mittel zum Messen des Schalls ein Geräuschmesser mit einem Geräuschaufnahmekanal (16) ist, der sich zur Spitze des Fühlers erstreckt und dafür ausgelegt ist, den Schall zu messen, der von der Hautprobe erzeugt wird, wenn der Fühler über eine Vertiefung oder Erhöhung auf der Haut streicht.

9. Vorrichtung nach Anspruch 8, umfassend einen Temperatursensor, der sich an einer Spitze des Fühlers befindet, um die Temperatur der Haut, die mit dem Fühler in Kontakt kommt, zu messen.

10. Vorrichtung nach Anspruch 9, umfassend einen Drucksensor, der sich an einer Spitze des Fühlers befindet und dafür ausgelegt ist, die Kraft anzuzeigen, die auf den Fühler ausgeübt wird, wenn er über die Haut streicht.

11. Vorrichtung nach Anspruch 10, wobei der Drucksensor einziehbar ist, um Änderungen des Profils der Haut aufgrund von Vertiefungen oder Erhöhungen auszugleichen.

12. Verfahren zum Lokalisieren von Läsionen des Weichgewebes, aufweisend:
(a) Bereitstellen eines Fühlers (10) mit Mitteln (14, 16) zum Messen des Feuchtigkeitsgehaltes eines Bereiches eines Hautgewebes und des Schalls, der aus diesem Bereich entsteht, und, optional, des Druckes jenes Bereiches, wenn der Fühler über den Hautbereich geführt wird;
(b) Führen des Fühlers über die Haut eines Patienten und Messen des Feuchtigkeitsgehaltes und des Schalls, der aus jenem Bereich entsteht und, optional, des Druckes des Bereiches.

13. Verfahren nach Anspruch 12, umfassend das Messen der Temperatur des Bereiches der Haut mit dem Fühler.

14. Verfahren nach Anspruch 12, wobei Schritt (b) periodisch wiederholt wird, um den Effekt einer Massage auf die Weichgewebeläsion zu bestimmen.

## Revendications

1. Appareil pour diagnostiquer la condition de la peau d'un être humain ou d'un animal, comprenant une sonde (10) pour contacter une zone souhaitée de la peau, qui comprend :
un moyen pour mesurer la teneur en eau de la peau au niveau de ladite zone (14),
**caractérisé en ce que** ladite sonde (10) comprend un moyen pour mesurer le son (16) produit par les tissus de la peau au niveau de ladite zone tandis que ladite sonde passe sur ladite zone.

2. Appareil selon la revendication 1, dans lequel une extrémité (18) de ladite sonde (10) est arrondie et suffisamment petite pour laisser une empreinte en creux en raison d'un oedème lorsqu'elle est pressée contre une zone endommagée de la peau.

3. Appareil selon la revendication 1, comprenant un moyen pour mesurer la température (12) des tissus de la peau au niveau de ladite zone.

4. Appareil selon la revendication 1, dans lequel ledit moyen pour mesurer la teneur en eau est un dispositif de mesure de résistance électrique (14) apte à mesurer la résistance électrique de la peau.

5. Appareil selon la revendication 1, dans lequel ledit moyen pour mesurer le son est un stéthoscope qui est incorporé dans ladite sonde comportant une chambre de réception de son (16) à proximité d'une extrémité (18) de ladite sonde (10).

6. Appareil selon la revendication 3, dans lequel ledit moyen pour mesurer la température comprend un thermocouple (12) situé à proximité d'une extrémité (18) de ladite sonde (10).

7. Appareil selon la revendication 1, comprenant un moyen pour mesurer la force appliquée sur la sonde (10) tandis que la sonde est passée sur la peau.

8. Appareil selon la revendication 1, dans lequel ledit moyen pour mesurer la teneur en eau est un dispositif de mesure de résistance électrique (14) ayant un contact au niveau d'une extrémité (18) de ladite sonde (10) apte à mesurer la résistance et, par conséquent, la teneur en eau de la peau au niveau de ladite zone, et ledit moyen pour mesurer le son est un sonomètre comportant un canal de réception de son (16) s'étendant jusqu'à l'extrémité de ladite sonde apte à mesurer le son produit par les tissus de la peau tandis que ladite sonde passe sur une lésion ou une protubérance sur la peau.

9. Appareil selon la revendication 8, comprenant un capteur de température situé à une extrémité de ladite sonde pour mesurer la température de la peau en contact avec ladite sonde.

10. Appareil selon la revendication 9, comprenant un capteur de pression situé à une extrémité de ladite sonde apte à indiquer la force appliquée sur ladite sonde tandis qu'elle passe sur la peau.

11. Appareil selon la revendication 10, dans lequel ledit capteur de pression est rétractable pour s'adapter aux changements de profil de la peau en raison de lésions et protubérances.

12. Procédé pour localiser des lésions des tissus mous, comprenant :
(a) la fourniture d'une sonde (10) comportant un moyen (14, 16) pour mesurer la teneur en eau d'une zone de tissus de la peau et le son provenant de ladite zone et, éventuellement, la pression de ladite zone tandis que ladite sonde est passée sur ladite zone de peau ;
(b) le passage de ladite sonde sur la peau d'un patient et la mesure de la teneur en eau et du son provenant de ladite zone et, éventuellement, la pression de ladite zone.

13. Procédé selon la revendication 12, comprenant la mesure de la température de ladite zone de la peau au moyen de la sonde.

14. Procédé selon la revendication 12, dans lequel l'étape (b) est répétée périodiquement pour déterminer l'effet d'un massage sur la lésion des tissus mous.
